(19) Europäisches Patentamt
European Patent Office
Office européen des brevets

(11) Numéro de publication: **0 193 475**
**A1**

(12) # DEMANDE DE BREVET EUROPEEN

(21) Numéro de dépôt: 86420029.0

(22) Date de dépôt: 30.01.86

(51) Int. Cl.⁴: C 07 C 45/76
C 07 C 45/57, C 07 C 45/46
C 07 C 49/84, C 07 C 49/782
C 07 C 49/813, C 07 C 49/807
C 07 C 49/76

(30) Priorité: 08.02.85 FR 8502007

(43) Date de publication de la demande:
03.09.86 Bulletin 86/36

(84) Etats contractants désignés:
AT BE CH DE GB IT LI LU NL SE

(71) Demandeur: RHONE-POULENC SPECIALITES CHIMIQUES
"Les Miroirs" 18, Avenue d'Alsace
F-92400 Courbevoie(FR)

(72) Inventeur: Crochemore, Michel
Domaine de Gilbertin 3, Rue des Lilas
F-69630 Chaponost(FR)

(72) Inventeur: Gay, Michel
11, Rue Garibaldi
F-69006 Lyon(FR)

(74) Mandataire: Vignally, Noel et al,
Rhône-Poulenc Interservices Service Brevets Chimie
Centre de Recherches de Saint-Fons B.P. 62
F-69192 Saint-Fons Cedex(FR)

(54) **Procédé d'acétoacétylation de composés aromatiques.**

(57) La présente invention concerne un nouveau procédé d'acétoacétylation de composés aromatiques ainsi que de nouveaux composés aromatiques comportant au moins 2 groupements acétoacétyles.

Le procédé consiste plus précisément à faire réagir le dicétène (ou l'un de ses dérivés halogénés, notamment fluorés) sur un composé aromatique, dans l'acide fluorhydrique et en présence d'un acide de Lewis ou d'un acide minéral protonique.

Les β-dicétones, les bis-β-dicétones et les poly β-dicétones qui sont ainsi obtenues ont des utilisations multiples telles que la complexation de métaux, la préparation de colorants ou la stabilisation des polymères hologénés.

EP 0 193 475 A1

Croydon Printing Company Ltd.

# PROCEDE D'ACETOACETYLATION DE COMPOSES AROMATIQUES.

La présente invention concerne un procédé d'acétoacétylation de composés aromatiques. Elle permet d'obtenir soit des ß-dicétones, soit des bis ß-dicétones, soit des poly-ß-dicétones.

La présente invention a également pour objet de nouveaux composés aromatiques comportant dans leur molécule au moins deux acétoacétyles.

Des procédés de préparation de ß-dicétones par acétoacétylation d'un composé aromatique à l'aide du dicétène sont connus.

Ainsi dans un article publié par A.B. BOESE dans Industrial and Engineering Chemistry (1940) n° 32, pages 20-21, il est décrit un procédé de préparation de benzoylacétone par réaction de benzène en fort excès et de dicétène en présence de chlorure d'aluminium. Dans ce procédé une grosse quantité de chlorure d'aluminium est nécessaire (2 moles pour 1 mole de dicétène) et la présence de chlorure d'aluminium (qui n'est pas récupéré) pose des problèmes d'isolement et de purification de la benzoylacétone formée.

Pour remédier à ces inconvénients la demande de brevet français n° 74/25 494 (publiée sous le n° 2 238 696) décrit un procédé de préparation de dicétones-1,3 aromatiques par réaction de fluorure d'acétoacétyle avec des composés aromatiques solides ou liquides, dans l'acide fluorhydrique à au moins 90 % et à des températures comprises entre -40° et +50°C. Ce procédé conduit à des aroylacétones, mais ne permet pas d'obtenir des bis-acétoacétylarènes.

Il a maintenant été trouvé un procédé d'acétoacétylation de composés aromatiques permettant d'obtenir à volonté, soit des aryl-1 butanedione-1,3, soit des arylène-1,1' bis (butanedione-1,3).

Lorsque l'on souhaite obtenir des aryl-1 butanedione-1,3, le procédé permet d'avoir une réaction plus rapide que dans l'art antérieur à température égale ou permet d'opérer sous des conditions plus douces, notamment à une température plus basse, que dans le procédé selon le brevet français n° 74/25 494. La possibilité d'effectuer la réaction à des températures plus basses permet d'acétoacétyler des composés

aromatiques pouvant comporter des groupements chimiques sensibles à la chaleur.

Plus précisément la présente invention consiste en un procédé d'acétoacylation de composé aromatique par le dicétène ou l'un de ses dérivés halogénés , en milieu essentiellement constitué par l'acide fluorhydrique, caractérisé en ce que l'on opère en présence d'un acide de Lewis ou d'un acide minéral protonique.

Dans le présent texte le terme composé aromatique englobe aussi bien les composés insaturés à doubles liaisons délocalisées comportant un ou plusieurs cycles benzéniques, éventuellement ortho- ou ortho- et péricondensés que les composés hétérocycliques dont les cycles comportent 5 ou 6 membres et possèdent des doubles liaisons délocalisées.

Par acides de Lewis on désigne conformément à la définition usuelle, des composés accepteurs de doublets électroniques, en excluant pour des raisons de commodité de différenciation les acides protoniques.

Pour la mise en oeuvre du procédé selon l'invention on pourra utiliser les différents types d'acides de Lewis et en particulier ceux qui sont cités dans l'ouvrage édité par G.A. OLAH 'Friedel-Crafts and related reactions" (1963) tome I, pages 191 à 197. Parmi ces acides de Lewis on emploie de préférence les halogénures acides (cf. G.A. OLAH Loc. cit. pages 201, 202 et 225 à 291) et en particulier les halogénures d'éléments tels que le bore, l'aluminium, l'étain, le phosphore, l'arsenic, le bismuth, l'antimoine, le titane, le zirconium, le vanadium, le molybdène, le fer, le cobalt, le nickel, le cuivre, le zinc et le cadmium.

Comme exemples spécifiques de tels halogénures on peut citer le trifluorure de bore, le tribromure de bore, le trichlorure de bore, le pentafluorure d'antimoine, le trifluorure d'antimoine, le chlorure stannique, le chlorure de zinc et le tétrachlorure de titane.

Comme acide minéral protonique on utilisera généralement l'acide sulfurique, l'acide chlorhydrique ou l'acide bromhydrique.

Parmi des raisons d'efficacité et de simplicité de mise en oeuvre ainsi que pour la facilité du traitement du mélange réactionnel final, le trifluorure de bore est tout particulièrement préféré.

L'acide fluorhydrique constitue pour l'essentiel le milieu dans

lequel s'opère la réaction d'acétoacétylation. Généralement le rapport pondéral entre l'acide fluorhydrique et le composé aromatique que l'on désire acétoacétyler est d'environ 1 à 20.

De préférence ce rapport pondéral varie de 3 à 10;

La quantité d'acide de Lewis ou d'acide minéral protonique servant de catalyseur peut varier dans de très larges limites. Si l'on exprime cette quantité par le rapport pondéral acide/HF elle est généralement comprise entre 0,01 et 1.

Lorsque l'acide utilisé est le trifluorure de bore, on utilise habituellement un rapport pondéral $BF_3$/HF compris entre 0,01 et 0,50 pour ne pas avoir une pression trop élevée. De préférence le rapport pondéral $BF_3$/HF se situe entre 0,02 et 0,2.

L'agent d'acétoacétylation est le plus fréquemment le dicétène. Ce peut être également l'un de ses dérivés halogénés tels que le fluorure d'acétoacétyle, qui peut se former par réaction in situ du dicétène avec l'acide fluorhydrique ou les halogénures de gamma-halogénoacétyle comme par exemple le fluorure de gamma-trifluoroacétoacétyle, le fluorure de gamma-trichloroacétoacétyle, le fluorure de gamma-dichloromonofluoroacétoacétyle, le fluorure de gamma-monochlorodifluoroacétoacétyle, le fluorure de gamma-difluoroacétoacétyle, le fluorure de gamma-dichloroacétoacétyle, le fluorure de gamma-monofluoroacétoacétyle, le fluorure de gamma-monochloroacétoacétyle.

La quantité d'agent d'acétoacétylation utilisée est variable en fonction du nombre de groupements acétoacétyle que l'on souhaite fixer sur le substrat aromatique. Le rapport molaire agent d'acétoacétylation/composé aromatique peut être inférieur à 1, car l'excès de composé aromatique peut alors être récupéré en fin de réaction et être éventuellement recyclé.

Mais généralement on utilise un rapport molaire agent d'acétoacétylation/composé aromatique de 1 à 3, bien que ces valeurs ne soient pas critiques.

Comme composé aromatique pouvant servir de substrat dans le procédé d'acétoacétylation conviennent tous les composés aromatiques mono- ou polycycliques, comportant éventuellement un ou plusieurs

hétéroatomes tels que S, O ou N dans le ou les cycles de leur molécule.

On peut citer à titre d'illustration :

- les composés aromatiques de formule générale (I) :

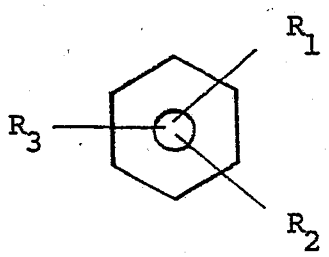

(I)

dans laquelle $R_1$, $R_2$ et $R_3$, identiques ou différents, représentent :

- . un atome d'hydrogène,
- . un radical alkyle, linéaire ou ramifié, ayant de 1 à 30 atomes de carbone,
- . un radical alkényle linéaire ou ramifié comportant 1 ou plusieurs doubles liaisons éthyléniques et ayant 2 à 20 atomes de carbone,
- . un radical alkoxy, linéaire ou ramifié, ayant de 1 à 12 atomes de carbone,
- . un atome d'halogène,
- . un groupement hydroxyle,
- . un radical alkyle linéaire ou ramifié ayant 2 à 30 atomes de carbone et comportant dans son enchaînement 1 ou plusieurs ponts - O -,
- . un groupement - O - $R_4$ - $COOR_5$ dans la formule duquel $R_4$ représente un radical alkylène linéaire ou ramifié ayant 1 à 6 atomes de carbone et $R_5$ représente un radical alkyle, linéaire ou ramifié, ayant 1 à 12 atomes de carbone,
- . un groupement - $COOR_6$, $R_6$ représentant un atome d'hydrogène ou un radical alkyle, linéaire ou ramifié ayant 1 à 20 atomes de carbone,
- . un groupement - CO - $R_6$, $R_6$ ayant les significations indiquées précédemment,
- . un groupement - O - CO - $R_7$, $R_7$ représentant un radical alkyle, linéaire ou ramifié, ayant 1 à 20 atomes de carbone,
- . un groupement cyclohexyle, cyclohexényle ou cyclohexadiényle,

. un groupement nitrile,

. un groupement 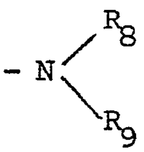 , $R_8$ et $R_9$, identiques ou différents, représentant un atome d'hydrogène ou un radical alkyle, linéaire ou ramifié, ayant de 1 à 12 atomes de carbone,

. un groupement $- NH - CO - O - R_{10}$, $R_{10}$ représentant un groupement alkyle, linéaire ou ramifié, ayant 1 à 12 atomes de carbone,

. un groupement $- S - R_8$, $R_8$ ayant les significations indiquées précédemment ;

- les composés aromatiques polycycliques ortho ou ortho- et péricondensés tels que par exemple le naphtalène, l'anthracène, l'indène, le phénalène, le phénanthrène, le pyrène, le naphtacène, le triphénylène, le pérylène dont chacun des cycles peut comporter 1 ou 2 substituants $R_1$ et $R_2$ tels que définis précédemment et dont une partie des cycles peut être partiellement ou totalement hydrogénée ;
- les composés aromatiques bicycliques de formule générale (II) :

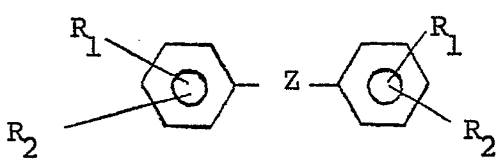

(II)

dans laquelle :

. Z représente : + un lien valentiel simple,
+ un groupement $- SO_2 -$,
+ un groupement $- SO -$,
+ un atome d'oxygène,
+ un atome de soufre,
+ un radical alkylène linéaire ou ramifié ayant 1 à 24 atomes de carbone,
+ un radical cycloalkylène,
+ un radical alkénylène linéaire ou ramifié comportant 1 ou 2 doubles liaisons et ayant 1 à 24 atomes de carbone,
+ un radical alkylène comportant de 6 à 20 atomes de

carbone, de 1 à 6 enchaînements constitués par - O -, - CO - ou - COO - et de 0 à 2 radicaux cyclo alkylène,

. $R_1$ et $R_2$, identiques ou différents possèdent des significations indiquées précédemment,

. l'un des cycles peut être partiellement ou totalement hydrogéné en cyclohexyle, cyclohexényle ou cyclohexadiényle ;

- les composés aromatiques de formule générale (III) :

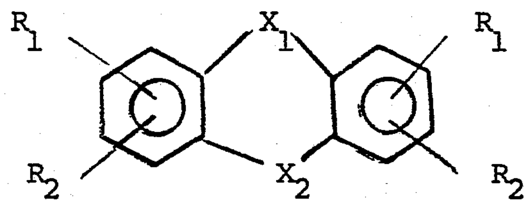

(III)

dans laquelle :

. les symboles $X_1$ et $X_2$ identiques ou différents représentent un lien valentiel simple, un atome d'oxygène ou atome de soufre ou un groupement méthylène,

. les symboles $R_1$ et $R_2$ identiques ou différents possèdent les significations indiquées précédemment,

. l'un des cycles peut être partiellement ou totalement hydrogéné ;

- les composés aromatiques hétérocycliques ayant un cycle ou 2 cycles condensés comportant 1 ou 2 hétéroatomes azote, soufre ou oxygène.

Parmi les composés aromatiques qui peuvent être utilisés comme substrats dans le procédé selon l'invention on préférera plus particulièrement :

- Les composés aromatiques de formule (I) dans laquelle $R_1$, $R_2$ et $R_3$, identiques ou différents, représentent :

. un atome d'hydrogène,

. un radical alkyle linéaire ou ramifié ayant de 1 à 12 atomes de carbone,

. un radical alkényle, linéaire ou ramifié, comportant 1 ou 2 doubles liaisons éthyléniques et ayant 2 à 12 atomes de carbone,

. un radical alkoxy, linéaire ou ramifié ayant 1 à 4 atomes de carbone,

. un atome de chlore, de fluor ou de brome,

. un groupement hydroxyle,

. un radical alkyle, linéaire ou ramifié, ayant de 2 à 12 atomes de carbone et comportant dans son enchaînement 1 ou 2 ponts - O -,

. un groupement - O - $R_4$ - $COOR_5$ dans la formule duquel $R_4$ représente un radical alkylène linéaire ou ramifié ayant 1 à 4 atomes de carbone et $R_5$ représente un radical alkyle linéaire ou ramifié ayant 1 à 8 atomes de carbone,

. un groupement - $COOR_6$ , dans la formule duquel $R_6$ représente un atome d'hydrogène ou un radical alkyle, linéaire ou ramifié, ayant 1 à 12 atomes de carbone,

. un groupement - CO - $R_6$ dans la formule duquel $R_6$ a les significations indiquées précédemment,

. un groupement - O - CO - $R_7$, dans la formule duquel $R_7$ représente un radical alkyle, linéaire ou ramifié, ayant 1 à 12 atomes de carbone,

. un groupement nitrile,

. un groupement 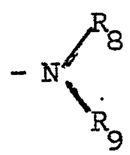 dans la formule duquel $R_8$ et $R_9$, identiques ou différents, représentent un atome d'hydrogène ou un radical alkyle linéaire ou ramifié ayant de 1 à 6 atomes de carbone,

. un groupement - NH - CO - O - $R_{10}$ dans la formule duquel $R_{10}$ représente un radical alkyle, linéaire ou ramifié ayant de 1 à 6 atomes de carbone,

. un groupement - S - $R_8$, dans la formule duquel $R_8$ a les significations indiquées précédemment,

- le naphtalène, le naphtalène comportant sur chacun de ses cycles condensés 1 ou 2 substituants $R_1$ et $R_2$ définis précédemment, le tétrahydro-1,2,3,4 naphtalène et le tétrahydro-1,2,3,4 naphtalène comportant sur chacun de ses cycles 1 ou 2 substituants $R_1$ et $R_2$ définis précédemment,

- les composés aromatiques bicycliques de formule générale (II) : dans laquelle :

. Z représente : + un lien valentiel simple

+ un groupement $- SO_2 -$
+ un groupement - SO -
+ un atome d'oxygène
+ un atome de soufre,
+ un radical alkylène linéaire ou ramifié ayant 1 à 12 atomes de carbone,
+ un radical
$- O - R_{11} - O - CO - R_{12} - CO - O - R_{13} - O -$
dans la formule duquel $R_{11}$, $R_{12}$ et $R_{13}$, identiques ou différents, représentent des radicaux alkylènes linéaires ou ramifiés ayant 1 à 12 atomes de carbone, $R_{12}$ représentant en outre un radical cyclohexylène ou cyclohexanediméthylène-1,4 ,
+ un radical
$- O - R_{11} - COO - R_{12} - OCO - R_{13} - O -$
dans la formule duquel $R_{11}$, $R_{12}$ et $R_{13}$, identiques ou différents, ont les significations indiquées précédemment,

. $R_1$ et $R_2$, identiques ou différents possèdent les significations indiquées précédemment,
. l'un des cycles peut être un groupement cyclohexyle ;

- les composés aromatiques de formule générale (III) dans laquelle :
. les symboles $X_1$ et $X_2$ identiques ou différents représentent un lien valentiel simple ou un atome d'oxygène,
. les symboles $R_1$ et $R_2$, identiques ou différents, possèdent les significations indiquées précédemment,
. l'un des cycles peut être un groupement cyclohexyle ;
- les composés aromatiques hétérocycliques ayant 1 ou 2 cycles condensés et comportant 1 atome d'azote, de soufre ou d'oxygène.

A titre d'exemples de composés de formule (I) on peut citer notamment le benzène, le toluène, le monochlorobenzène, le méthoxybenzène, l'éthoxybenzène, l'orthoxylène, le métaxylène, le paraxylène, le monobromobenzène, le monofluorobenzène, le triméthyl-1,3,5 benzène, le triméthyl-1,2,4 benzène, le triméthyl-1,2,3 benzène, le triéthyl-1,2,4 benzène, le triéthyl-1,3,5 benzène, le diméthoxy-1,2

benzène, le diméthoxy-1,3 benzène, le diméthoxy-1,4 benzène, le difluoro-1,2 benzène, le difluoro-1,3 benzène, le difluoro-1,4 benzène, le dichloro-1,2 benzène, le dichloro-1,3 benzène, le dichloro-1,4 benzène, le phénoxy-2 acétate d'éthyle, le phénoxy-3 propionate d'éthyle, le phénoxy-4 butyrate d'éthyle, le phénoxy-2 acétate de butyle, le phénol, le méthoxy-2 phénol, le méthoxy-4 phénol, le benzoate de méthyle, le benzoate de butyle, le benzoate d'éthyle, le benzoate d'hexyle, le benzoate de dodécyle, l'acétophénone, la butyrophénone, l'hexanophénone, l'acétate de phényle, le propionate de phényle, le butyrate de phényle, l'octanoate de phényle, le benzonitrile, le diméthylaminobenzène, le diéthylaminobenzène, l'aniline, le phénylcarbamate d'éthyle, le phénylcarbamate de butyle, le phénylcarbamate d'hexyle, le thiophénol, le méthylthiobenzène, l'éthylthiobenzène, le butylthiobenzène.

A titre d'exemples de composés aromatiques polycycliques condensés on peut citer notamment le naphtalène, le tétrahydro-1,2,3,4 naphtalène, le méthyl-1 naphtalène, le méthyl-2 naphtalène, l'éthyl-1 naphtalène, l'éthyl-2 naphtalène, le méthoxy-1 naphtalène, le méthoxy-2 naphtalène, l'éthoxy-1 naphtalène, l'éthoxy-2 naphtalène, le naphtol-1, le naphtol-2, le diméthyl-1,4 naphtalène, le diméthyl-2,3 naphtalène, le diméthoxy-1,4 naphtalène, le diméthoxy-1,3 naphtalène, le diméthoxy-1,2 naphtalène, le diméthoxy-2,3 naphtalène, le propoxy-1 naphtalène, le propoxy-2 naphtalène, le butoxy-1 naphtalène, le butoxy-2 naphtalène, le chloro-1 naphtalène, le chloro-2 naphtalène, le fluoro-1 naphtalène, le fluoro-2 naphtalène, le dichloro-1,2 naphtalène, le dichloro-1,3 naphtalène, le dichloro-1,4 naphtalène, le dichloro-1,5 naphtalène, le dichloro-1,6 naphtalène, le dichloro-1,7 naphtalène, le dichloro-1,8 naphtalène, le dichloro-2,3 naphtalène, le dichloro-2,6 naphtalène, le dichloro-2,7 naphtalène, le triméthyl-1,2,5 naphtalène, le triméthyl-1,2,6 naphtalène, le triméthyl-1,2,7 naphtalène, le triméthyl-2,3,6 naphtalène, le diméthyl-1,6 isopropyl-4 naphtalène, l'acétonaphtone, la propionaphtone, la butyronaphtone.

A titre d'exemples de composés aromatiques de formule (II) on peut notamment citer le biphényle, le diphénylméthane, le diphényl-2,2 propane, le diphényl-1,3 diméthyl-2,2 pentane, l'oxyde de diphényle, le diphényl-1,6 hexane, le diphényl-1,8 octane, le diphényl-1,10 décane, le

diphénylsulfoxyde, la diphénylsulfone, le sulfure de diphényle, le di(phénoxy-2 acétate) d'éthylèneglycol, le di(phénoxy-2 acétate) de butanediol-1,4, le di(phénoxy-2 acétate) de diméthyl-2,2 propanediyle, le di(phénoxy-3 propionate) d'éthylèneglycol, le succinate de di-(phénoxy-2 éthyle), l'adipate de di(phénoxy-2 éthyle), le sébaçate de di(phénoxy-2 éthyle), le succinate de di(phénoxy-3 propyle), l'adipate de di(phénoxy-3 propyle), le succinate de di(phénoxy-4 butyle), l'adipate de di(phénoxy-4 butyle), le di(phénoxy-2 acétate) de cyclohexanediméthylène-1,4.

A titre d'exemples de composés aromatiques de formule (III), on peut citer notamment le dibenzo-p-dioxane, le dibenzofuranne, le biphénylène et le fluorène.

A titre d'exemples de composés aromatiques hérérocycliques on peut citer notamment la pyridine, le thiophène, le furanne, le pyrrole, la quinoléine, l'isoquinoléine.

La température à laquelle est conduit le procédé selon l'invention varie généralement entre -40°C et +50°C. De préférence cette température se situe entre -30°C et +30°C.

La pression sous laquelle est réalisée la réaction d'acétoacétylation est généralement la pression autogène due essentiellement à l'acide fluorhydrique et à l'acide de Lewis ou l'acide minéral protonique, lorsque cesdits acides sont normalement à l'état gazeux à la température de la réaction. Il en est notamment ainsi dans une variante préférée de l'invention mettant en oeuvre le trifluorure de bore.

La durée de la réaction d'acétoacétylation est variable en fonction de la température, du composé aromatique mis en oeuvre et du nombre d'acétoacétylations à effectuer sur le composé aromatique.

Généralement cette durée se situe entre 30 minutes et plusieurs heures.

Compte-tenu des faibles températures et des pressions généralement peu élevées utilisées pour la mise en oeuvre du procédé selon l'invention, on peut utiliser un appareillage en matière plastique telle que le polyéthylène, le polypropylène ou le polychlorure de vinyle. On peut bien entendu opérer dans un appareillage en métal, le plus souvent en acier inoxydable, en particulier lorsque la pression dépasse

1,5 bar.

Pour mettre en oeuvre le procédé selon l'invention on peut par exemple charger en quelques minutes l'acide fluorhydrique et le dicétène (ou son dérivé), dans un réacteur en acier inoxydable à une température inférieure à la température de réaction choisie, par exemple inférieure ou égale à 0°C. On introduit aussi l'acide de Lewis ou l'acide minéral protonique ; dans une variante préférée mettant en oeuvre le trifluorure de bore, celui-ci crée une pression dans l'appareil. On ajoute alors le composé aromatique en quelques minutes généralement. On peut rajouter éventuellement une nouvelle quantité d'acide de Lewis ou d'acide minéral protonique. On ajuste la température à la valeur désirée et on maintient un certain temps, généralement quelques heures, à cette température.

L'ordre d'introduction des différents composés peut évidemment être modifiée. Le dicétène (ou son dérivé) peut par exemple être introduit après les autres réactifs et catalyseurs, sans modification fondamentale du procédé selon l'invention.

Le traitement de la masse réactionnelle finale peut consister à neutraliser l'acide fluorhydrique après avoir mis l'essai en milieu aqueux (par coulée dans la glace par exemple). La ß-dicétone, la bis ß-dicétone ou la poly ß-dicétone obtenue peut être extraite par un solvant adapté.

On peut également distiller l'acide fluorhydrique pour le recycler dans une autre opération et traiter le résidu contenant le produit de la réaction selon les techniques habituelles de séparation : recristallisation, distillation, extraction.

Comme cela a été indiqué précédemment la présente invention a également pour objet de nouveaux composés aromatiques comportant dans leur molécule au moins 2 motifs acétoacétyle.

Ce sont plus particulièrement les composés aromatiques bis-ß-dicétoniques ou poly ß-dicétoniques

- de formule générale (IV) :

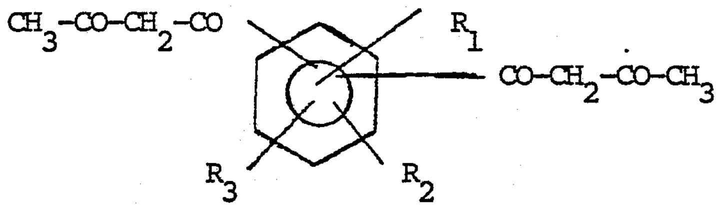 (IV)

dans laquelle $R_1$, $R_2$ et $R_3$ identiques ou différents représentent :

+ un atome d'hydrogène ;
+ un radical alkyle linéaire ou ramifié ayant 1 à 12 atomes de carbone ;
+ un radical alkoxy linéaire ou ramifié ayant 1 à 4 atomes de carbone ;
+ un atome de chlore ou de brome ;

- de formule générale (V) :

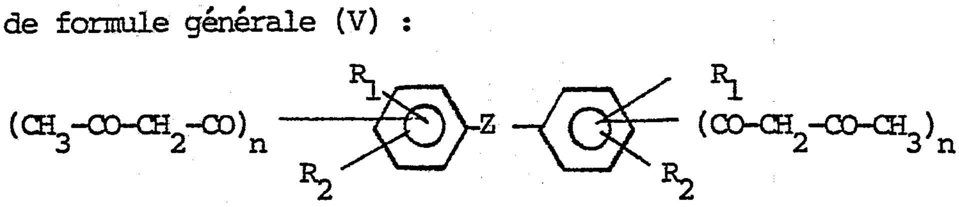 (V)

dans laquelle :

+ $R_1$ et $R_2$ identiques ou différents représentent :
  . un atome d'hydrogène
  . un groupement alkyle linéaire ou ramifié ayant 1 à 12 atomes de carbone,
  . un groupement alkoxy linéaire ou ramifié ayant 1 à 4 atomes de carbone,
  . un atome de chlore ou de brome.
+ Z représente un lien valentiel
  . un atome d'oxygène ou de soufre,
  . un radical alkylène linéaire ou ramifié ayant 1 à 12 atomes de carbone,
+ n représente 1 ou 2.

A titre d'exemples de composés de formules (IV) et (V) on peut citer :

- l'oxyde de bis (acétoacétyl-4 phényle) ;
- le diacétoacétyl-1,2 diméthoxy-4,5 benzène ;
- le diacétoacétyl-1,3 triméthyl-2,4,6 benzène ;

- le diacétoacétyl-4,4' diphénylméthane.

Les ß-dicétones, bis ß-dicétones ou poly ß-dicétones obtenues par le procédé selon l'invention ont des multiples utilisations connues telles que par exemple la complexation des métaux ou la préparation de colorants. Une autre application intéressante de certaines ß-dicétones, bis-ß-dicétones ou poly ß-dicétones concerne la stabilisation des polymères halogénés et notamment du polychlorure de vinyle.

Exemples 1 à 3 et essais comparatifs A et B

Dans un réacteur en acier inoxydable de 50 cm3 avec agitation, on charge de l'acide fluorhydrique et du dicétène en 15 minutes à 0°C (les quantités de HF et de dicétène utilisées pour les différents exemples sont indiquées dans le tableau I ci-après).

On introduit alors du trifluorure de bore (1 bar de pression) : environ 1,4 g au total : partie dissoute + partie gazeuse.

Ensuite on rajoute en 15 minutes à 0°C de l'oxyde de diphényle.

On maintient 1 bar de pression de $BF_3$.

Puis on laisse remonter la température du mélange réactionnel en 15 minutes à 10°C et on maintient un certain temps (indiqué dans le tableau I) à cette température.

On utilise le même mode opératoire pour les essais comparatifs A et B (selon l'art antérieur le plus proche) avec les différences suivantes :

- introduction des réactifs à 10°C
- absence de trifluorure de bore
- température de réaction de 20°C ou 30°C au lieu de 10°C.

Le traitement de la masse réactionnelle finale est le même pour les exemples 1 à 3 et pour les essais comparatifs A et B, à l'exception du dégazage du trifluorure de bore qui n'est présent que dans les exemples.

La masse réactionnelle est coulée dans 130 g de glace. Le réacteur est ensuite lavé par 3 fois 30cm3 d'un mélange à 50/50 en volume de dichlorométhane et d'éther isopropylique.

L'acide fluorhydrique est neutralisé par du carbonate de potassium.

On sépare la phase organique, puis on effectue une extraction de la phase aqueuse par 50 cm3 de mélange de dichlorométhane/éther isopropylique. On réunit les deux solutions organiques, on sèche à l'aide de chlorure de calcium anhydre, puis on élimine les solvants par distillation. On détermine par chromatographie en phase gazeuse couplée à un spectromètre de masse et par résonance magnétique nucléaire :

- le taux de transformation (TT) de l'oxyde de diphényle ;
- le rendement (RT) en oxyde de bis (acétoacétyl-4 phényle) pour les exemples 1 à 3 (selon l'invention) par rapport à l'oxyde de diphényle transformé ;
- le rendement (RT) en acétoacétyl-1 phénoxy-4 benzène (solide blanc fondant à 73°C) pour les essais comparatifs (selon l'art antérieur) par rapport à l'oxyde de diphényle transformé.

Le tableau I ci-après rassemble les caractéristiques principales et les résultats des différents essais décrits précédemment.

On constate que l'on obtient une bis-ß-dicétone avec le procédé selon l'invention alors que le procédé le plus proche de l'art antérieur conduit à la ß-dicétone, en utilisant des rapports molaires dicétène/composé aromatique supérieurs à 2 dans tous les cas, et malgré une température plus élevée et une durée de réaction plus longue dans les essais comparatifs A et B.

TABLEAU I

| ESSAIS | oxyde de diphényle (poids en g) | HF (poids en g) | $BF_3$ (poids en g) | Rapport molaire dicétène/oxyde de diphényle | Rapport pondéral $BF_3/HF$ | température | Durée (heures) | TT % | RT % |
|---|---|---|---|---|---|---|---|---|---|
| exemple 1 | 4 | 20 | 1,4 | 2,2 | 0,07 | + 10°C | 4 | 100 | 83 (bis) |
| exemple 2 | 6 | 30 | 1,4 | 2,3 | 0,07 | + 10°C | 4 | 100 | 76 (bis) |
| exemple 3 | 6 | 30 | 1,4 | 2,3 | 0,07 | + 10°C | 2 | 100 | 83 (bis) |
| essai comparatif A | 4 | 20 | 0 | 2,5 | 0 | + 20°C | 16 | 100 | 95 mono |
| essai comparatif B | 4 | 20 | 0 | 2,5 | 0 | + 30°C | 16 | 100 | 90 mono |

Exemples 4 à 7 et essais comparatifs C, D et E

En opérant comme cela a été décrit dans les exemples 1 à 3 on applique le procédé selon l'invention à 3 autres composés aromatiques :

- le diméthoxy-1,2 benzène (DMB) (exemple 4)
- le triméthyl-1,3,5 benzène (TMB) (exemple 5)
- le diphénylméthane (DPM) (exemples 6 et 7)

De la même façon en appliquant le procédé de l'art antérieur comme cela a été décrit pour les essais comparatifs A et B, 3 essais comparatifs C, D et E sont réalisés, respectivement avec le triméthyl-1,3,5 benzène (C) et le diphénylméthane (D et E).

Le tableau II ci-après rassemble les caractéristiques principales de ces exemples et essais comparatifs, ainsi que les taux de transformation (TT) des composés aromatiques et les rendements (RT) en bis-ß-dicétones et en mono-ß-dicétones obtenues par rapport aux composés aromatiques transformés.

Les bis-ß-dicétones et les mono-ß-dicétones ont été caractérisées par les méthodes d'analyses indiquées pour les exemples 1 à 3.

Ce sont :

- le diacétoacétyl-1,2 diméthoxy-4,5 benzène pour l'exemple 4 (solide blanc fondant à 50°C) ;
- le diacétoacétyl-1,3 triméthyl-2,4,6 benzène pour l'exemple 5 (solide blanc fondant à 122°C) ;
- le diacétoacétyl-4, 4' diphénylméthane pour les exemples 6 et 7 (solide blanc fondant à 60°C°) ;
- l'acétoacétyl-1 triméthyl-2,4,6 benzène (essai C) (solide blanc fondant à moins de 50°C) ;
- l'(acétoacétyl-4 phényl)- phénylméthane (essais D et E).

TABLEAU II

| ESSAIS | composé aromatique (en moles) | HF (poids en g) | $BF_3$ (poids en g) | Rapport molaire dicétène/composé aromatique | Rapport pondéral $BF_3/HF$ | température | Durée (heures) | TT % | RT % |
|---|---|---|---|---|---|---|---|---|---|
| exemple 4 | DMB (0,030) | 20 | 1,4 | 2,2 | 0,07 | + 20°C | 4 | 100 | 50 (bis) |
| exemple 5 | TMB (0,030) | 20 | 1,4 | 2,2 | 0,07 | + 10°C | 4 | 100 | 93 (bis) |
| exemple 6 | DPM (0,024) | 20 | 1,4 | 2,2 | 0,07 | - 10°C | 2,5 | 40 | 100 (bis) |
| exemple 7 | DPM (0,024) | 20 | 1,4 | 2,2 | 0,07 | + 10°C | 4 | 100 | 53 (bis) |
| essai comparatif C | TMB (0,030) | 20 | 0 | 1,1 | 0 | + 20°C | 6 | 100 | 97 (mono) |

TABLEAU II (fin)

| ESSAIS | composé aromatique (en moles) | HF (poids en g) | $BF_3$ (poids en g) | Rapport molaire dicétène/composé aromatique | Rapport pondéral $BF_3/HF$ | température | Durée (heures) | TT % | RT % |
|---|---|---|---|---|---|---|---|---|---|
| essai comparatif D | DPM (0,030) | 20 | 0 | 1,5 | 0 | + 20°C | 64 | 45 | 30 (mono) |
| essai comparatif E | DPM (0,024) | 20 | 0 | 2,2 | 0 | - 10°C | 3,5 | 34 | 9 (mono) |

Exemple 8

En opérant comme cela a été décrit pour les exemples 1 à 3 on répète l'acétoacétylation du triméthyl-1,3,5 benzène (TMB) effectuée dans l'exemple 5 mais en utilisant :

- un rapport molaire dicétène/TMB de 3,6 (au lieu de 2,2 pour l'exemple 5),
- une température de 20°C (au lieu de 10°C pour l'exemple 5).

L'essai dure 4 heures.

On obtient les résultats suivants :

- TT du triméthyl-1,3,5 benzène : 100 %
- RT en dicétoacétyl-1,3 triméthyl-2,4,6 benzène : 100 %

Exemples 9 à 11 et essais comparatifs F, G et H

En opérant comme cela a été décrit pour les exemples 1 à 3, on effectue

a) l'acétoacétylation du diphénylène (DPL)
  - d'une part selon le procédé de l'invention, dans HF en présence de $BF_3$ : exemple 9
  - d'autre part à titre de comparaison dans HF en l'absence de $BF_3$ : essai comparatif F.

b) l'acétoacétylation du sulfure de diphényle (SDP)
  - d'une part dans HF + $BF_3$ : exemple 10
  - d'autre part dans HF seul : essai comparatif G

c) l'acétoacétylation du N-phénylcarbamate d'éthyle (PCE) :
  - d'une part dans HF + $BF_3$ : exemple 11 ;
  - d'autre part dans HF seul : essai comparatif H.

L'exemple 9 et l'essai comparatifs F conduisent tous deux à l'acétoacétyl-4 diphénylène, mais avec un rendement beaucoup plus élevé pour une durée beaucoup plus faible avec le procédé selon la présente invention.

L'exemple 19 conduit à l'acétoacétyl-4 diphénylsulfure (solide blanc fondant à 114°C) tandis que dans l'essai comparatif G sans $BF_3$, il n'y a pas d'acétoacétylation.

L'exemple 11 conduit à l'acétoacétyl-4 phénylcarbamate d'éthyle tandis que dans l'essai comparatif H sans $BF_3$, il n'y a pas

d'acétoacétylation.

Le tableau III ci-après rassemble les caractéristiques principales de ces exemples et essais comparatifs, ainsi que les TT des composés aromatiques et les RT en composés ß-dicétoniques.

TABLEAU III

| ESSAIS | composé aromatique (en moles) | HF (poids en g) | $BF_3$ (poids en g) | Rapport molaire dicétène/composé aromatique | Rapport pondéral $BF_3/HF$ | température | Durée (heures) | TT % | RT % |
|---|---|---|---|---|---|---|---|---|---|
| exemple 9 | DPL (0,024) | 20 | 1,4 | 2,2 | 0,07 | + 10°C | 4 | 92 | 77 (mono) |
| exemple 10 | SDP (0,025) | 20 | 1,4 | 2,4 | 0,07 | + 20°C | 16 | 90 | 90 (mono) |
| exemple 11 | PCE (0,028) | 20 | 1,4 | 1,2 | 0,07 | + 20°C | 16 | 60 | 30 (mono) |
| essai comparatif F | DLP (0,024) | 20 | 0 | 2,2 | 0 | + 20°C | 16 | 40 | 27 (mono) |
| essai comparatif G | SDP (0,025) | 20 | 0 | 2,4 | 0 | + 20°C | 16 | 0 | 0 |
| essai comparatif H | PCE (0,028) | 20 | 0 | 1,2 | 0 | + 20°C | 16 | 0 | 0 |

Exemples 12 à 17

En opérant comme cela a été décrit pour les exemples 1 à 3, on effectue l'acétoacétylation de l'oxyde de diphényle (DPO) dans HF en présence comme catalyseur de différents acides de Lewis ou d'un acide minéral protonique ($H_2SO_4$ à 96-98 %).

On obtient dans tous les exemples de l'oxyde de bis(acétoacétyl-4 phényle) qui est un solide blanc fondant à 152°C.

Le tableau IV ci-après rassemble les caractéristiques principales de ces exemples, ainsi que les TT et RT obtenus.

TABLEAU IV

| ESSAIS | oxyde de diphényle (en moles) | HF (poids en g) | catalyseur (poids en g) | Rapport molaire dicétène/oxyde de diphényle | Rapport pondéral catalyseur/HF | température | Durée (heures) | TT % | RT % |
|---|---|---|---|---|---|---|---|---|---|
| exemple 12 | 0,024 | 20 | $H_2SO_4$ 3,0 | 2,5 | 0,15 | + 10°C | 4 | 100 | 26 (bis) |
| exemple 13 | 0,024 | 20 | $TiCl_4$ 3,0 | 2,5 | 0,15 | + 10°C | 4 | 100 | 43 (bis) |
| exemple 14 | 0,024 | 20 | $SnCl_4$ 3,0 | 2,5 | 0,15 | + 10°C | 4 | 100 | 46 (bis) |
| exemple 15 | 0,024 | 20 | $ZnCl_2$ 3,0 | 2,5 | 0,15 | + 10°C | 4 | 100 | 48 (bis) |
| exemple 16 | 0,024 | 20 | $SbF_5$ 3,0 | 2,5 | 0,15 | + 10°C | 4 | 100 | 52 (bis) |
| exemple 17 | 0,024 | 20 | $SbF_3$ 3,0 | 2,5 | 0,15 | + 10°C | 4 | 100 | 55 (bis) |

Exemple 18 et essai comparatif J

En opérant comme cela a été décrit pour les exemples 1 à 3, on effectue l'acétoacétylation du monochlorobenzène

a) d'une part selon le procédé de l'invention dans HF en présence de pentafluorure d'antimoine (exemple 18) :

- 0,030 mole de monochlorobenzène
- 20 g de HF
- 12 g de $SbF_5$
- rapport molaire dicétène/monochlorobenzène = 1,2
- température : + 20°C
- durée : 16 heures.

On obtient les résultats suivants :

TT = 100 %

RT en acétoacétyl-1 chloro-4 benzène : 100 % (solide blanc fondant à 70°C)

b) d'autre part à titre de comparaison dans les mêmes conditions et avec les mêmes charges, mais sans Sb F5 (essai J).

On obtient les résultats suivants :

TT : 0 %

RT : 0 %

Exemples 19 à 23 et essais comparatifs K, L, M, N et P

En opérant comme cela a été décrit pour les exemples 1 à 3, on effectue l'acétoacétylation de différents composés aromatiques :

- l'adipate de di(phénoxy-2 éthyle (ADPE)
- le di(phénoxyacétate) d'éthylèneglycol (PAEG)

$C_6H_5\ O-CH_2-COOCH_2-CH_2-OCO-CH_2-OC_6H_5$

- le di(phénoxyacétate)de diméthyl-2,2 propanediyle (PADP) :

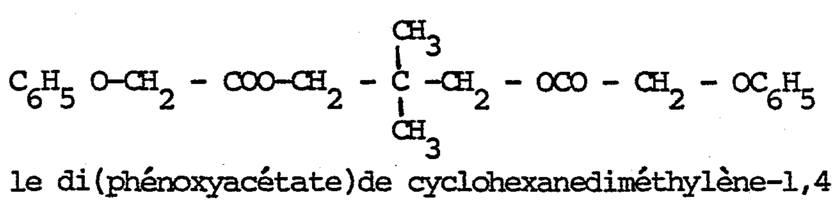

- le di(phénoxyacétate)de cyclohexanediméthylène-1,4 (PACD)

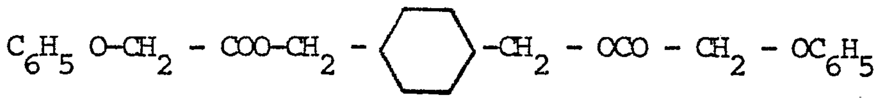

- le phénoxyacétate d'éthyle (PAE)

a) d'une part selon le procédé de l'invention, dans HF en présence de $BF_3$ : exemples 19 à 23 ;

b) d'autre part à titre de comparaison dans HF en l'absence de catalyseur : essais comparatifs K, L, M, N, P.

Les exemples et les essais comparatifs conduisent respectivement aux composés suivants :

- exemple 19 et essai comparatif K à :
  l'adipate de di(acétoacétyl-4 phénoxy-2 éthyle)
- exemple 20 et essai comparatif L au :
  di(acétoacétyl-4 phénoxy acétate) d'éthylène glycol (solide blanc fondant à 120°C).
- exemple 21 et essai comparatif M au :
  di(acétoacétyl-4 phénoxyacétate) de diméthyl-2,2 propanediyle
- exemple 22 et essai comparatif N au :
  di(acétoacétyl-4 phénoxyacétate) de cyclohexanediméthylène-1,4 (solide blanc fondant à 162°C)
- exemple 23 et essai comparatif P au :
  acétoacétyl-4 phénoxyacétate d'éthyle (solide blanc fondant à 70-71°C).

Le tableau V ci-après rassemble les caractéristiques principales de ces différents essais, ainsi que les TT et RT obtenus. On constate que les exemples effectués selon l'invention permettent d'obtenir une réaction d'acétoacétylation beaucoup plus rapide dans les mêmes conditions que les essais effectués sans catalyseur.

TABLEAU V

| ESSAIS | composé aromatique ( en moles ) | HF (poids en g) | $BF_3$ (poids en g) | Rapport molaire dicétène/composé aromatique | Rapport pondéral $BF_3/HF$ | température | Durée (heures) | TT % | RT % |
|---|---|---|---|---|---|---|---|---|---|
| exemple 19 | ADPE 0,015 | 20 | 1,4 | 2,2 | 0,07 | + 10°C | 4 | 100 | 95 (bis) |
| essai comparatif K | ADPE 0,015 | 20 | 0 | 2,2 | 0 | + 20°C | 16 | 100 | 91 (bis) |
| exemple 20 | PAEG 0,015 | 20 | 1,4 | 2,2 | 0,07 | + 10°C | 4 | 100 | 86 (bis) |
| essai comparatif L | PAEG 0,015 | 20 | 0 | 2,2 | 0 | + 20°C | 16 | 100 | 80 (bis) |
| exemple 21 | PADP 0,014 | 20 | 1,4 | 2,35 | 0,07 | + 10°C | 4 | 100 | 100 (bis) |
| essai comparatif M | PADP 0,014 | 20 | 0 | 2,35 | 0 | + 20°C | 16 | 100 | 100 (bis) |

TABLEAU V (Fin)

| ESSAIS | composé aromatique ( en moles ) | HF (poids en g) | $BF_3$ (poids en g) | Rapport molaire dicétène/composé aromatique | Rapport pondéral $BF_3/HF$ | température | Durée (heures) | TT % | RT % |
|---|---|---|---|---|---|---|---|---|---|
| exemple 22 | PACD 0,013 | 20 | 1,4 | 2,5 | 0,07 | + 10°C | 4 | 100 | 80 (bis) |
| essai comparatif N | PACD 0,013 | 20 | 0 | 2,5 | 0 | + 20°C | 16 | 100 | 76 (bis) |
| exemple 23 | PAE 0,028 | 20 | 1,4 | 1,14 | 0,07 | 0°C | 4 | 100 | 86 (mono) |
| essai comparatif P | PAE 0,028 | 20 | 0 | 1,14 | 0 | + 20°C | 16 | 100 | 85 (mono) |

Exemple 24

En opérant comme cela a été décrit pour les exemples 1 à 3, on effectue l'acétoacétylation du monochlorobenzène :

- 0,035 mole de monochlorobenzène
- 20 g de HF
- 1,4 g de $BF_3$
- rapport molaire dicétène/monochlorobenzène = 1,34
- température : 10°C
- durée : 4 heures.

On obtient les résultats suivants :

TT = 100 %

RT en acétoacétyl-1 chloro-4 benzène : 100 %

Exemple 25 et Essai comparatif R

En opérant comme cela a été décrit pour les exemples 1 à 3, on effectue l'acétoacétylation du phényldodécane :

- 0,02 mole de phényldodécane
- 20 g de HF
- 1,4 g de $BF_3$
- rapport molaire dicétène/phényldodécane = 1,5
- température : 10°C
- durée : 4 heures.

On obtient les résultats suivants :

TT = 100 %

RT en (acétoacétyl-4 phényl) dodécane : 85 %

L'(acétoacétyl-4 phényl) dodécane est un solide blanc ayant un point de fusion de 52°C

Selon le même mode opératoire on effectue l'essai comparatif R avec les mêmes charges de phényldodécane, de dicétène et de HF, mais sans BF3.

Les conditions sont les suivantes :

- température : 20°C
- durée : 16 heures.

On obtient les résultats suivants : aucune transformation du phényldodécane.

# REVENDICATIONS

1) Procédé d'acétoacétylation de composé aromatique par le dicétène ou l'un des ses dérivés halogénés, en milieu essentiellement constitué par l'acide fluorhydrique, caractérisé en ce que l'on opère en présence d'un acide de Lewis ou d'un acide minéral protonique.

2) Procédé selon la revendication 1 caractérisé en ce que l'acide de Lewis utilisé est un halogénure d'un élément tel que le bore, l'aluminium, l'étain, le phosphore, l'arsenic, le bismuth, l'antimoine, le titane, le zirconium, le vanadium, le molybdène, le fer, le cobalt, le nickel, le cuivre, le zinc et le cadmium.

3) Procédé selon les revendications 1 ou 2 caractérisé en ce que l'acide de Lewis utilisé est choisi parmi le trifluorure de bore, le tribromure de bore, le trichlorure de bore, le pentafluorure d'antimoine, le trifluorure d'antimoine, le tétrachlorure de titane, le chlorure stannique et le chlorure de zinc.

4) Procédé selon la revendication 1 caractérisé en ce que l'acide minéral protonique utilisé est choisi parmi l'acide sulfurique, l'acide chlorhydrique et l'acide bromhydrique.

5) Procédé selon l'une des revendications 1 à 3 caractérisé en ce que l'acide de Lewis utilisé est le trifluorure de bore.

6) Procédé selon l'une des revendications 1 à 5 caractérisé en ce que l'agent d'acétoacétylation est introduit sous forme de dicétène.

7) Procédé selon l'une des revendications 1 à 6 caractérisé en ce que le rapport pondéral acide de Lewis ou acide minéral protonique/acide fluorhydrique est compris entre 0,01 et 1.

8) Procédé selon la revendication 5 caractérisé en ce que le rapport pondéral trifluorure de bore/acide fluorhydrique est compris entre 0,01 et 0,5 et de préférence entre 0,02 et 0,2.

9) Procédé selon l'une des revendications 1 à 8 caractérisé en ce que le composé aromatique est choisi parmi :

- les composés aromatiques de formule générale (I) :

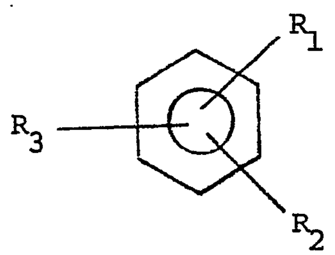

(I)

dans laquelle $R_1$, $R_2$ et $R_3$, identiques ou différents, représentent :

. un atome d'hydrogène,

. un radical alkyle, linéaire ou ramifié, ayant de 1 à 30 atomes de carbone,

. un radical alkényle linéaire ou ramifié comportant 1 ou plusieurs doubles liaisons éthyléniques et ayant 2 à 20 atomes de carbone,

. un radical alkoxy, linéaire ou ramifié, ayant de 1 à 12 atomes de carbone,

. un atome d'halogène,

. un groupement hydroxyle,

. un radical alkyle linéaire ou ramifié ayant 2 à 30 atomes de carbone et comportant dans son enchaînement 1 ou plusieurs ponts - O -,

. un groupement - O - $R_4$ - $COOR_5$ dans la formule duquel $R_4$ représente un radical alkylène linéaire ou ramifié ayant 1 à 6 atomes de carbone et $R_5$ représente un radical alkyle, linéaire ou ramifié, ayant 1 à 12 atomes de carbone,

. un groupement - $COOR_6$, $R_6$ représentant un atome d'hydrogène ou un radical alkyle, linéaire ou ramifié ayant 1 à 20 atomes de carbone,

. un groupement - CO - $R_6$, $R_6$ ayant les significations indiquées précédemment,

. un groupement $- O - CO - R_7$, $R_7$ représentant un radical alkyle, linéaire ou ramifié, ayant 1 à 20 atomes de carbone,

. un groupement cyclohexyle, cyclohexényle ou cyclohexadiényle,

. un groupement nitrile,

. un groupement - 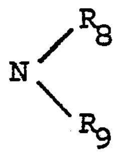 , $R_8$ et $R_9$, identiques ou différents, représentant un atome d'hydrogène ou un radical alkyle, linéaire ou ramifié, ayant de 1 à 12 atomes de carbone,

. un groupement $- NH - CO -O- R_{10}$, $R_{10}$ représentant un groupement alkyle, linéaire ou ramifié, ayant 1 à 12 atomes de carbone,

. un groupement $- S - R_8$, $R_8$ ayant les significations indiquées précédemment ;

- les composés aromatiques polycycliques ortho ou ortho- et péricondensés tels que par exemple le naphtalène, l'anthracène, l'indène, le phénalène, le phénanthrène, le pyrène, le naphtacène, le triphénylène, le pérylène dont chacun des cycles peut comporter 1 ou 2 substituants $R_1$ et $R_2$ tels que définis précédemment et dont une partie des cycles peut être partiellement ou totalement hydrogénée ;

- les composés aromatiques bicycliques de formule générale (II) :

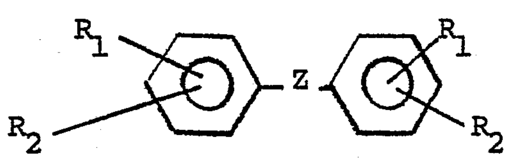 (II)

dans laquelle :

. Z représente : + un lien valentiel simple,

+ un groupement $- SO_2 -$,

+ un groupement $- SO -$,

+ un atome d'oxygène,

+ un atome de soufre,

+ un radical alkylène linéaire ou ramifié ayant 1 à 24 atomes de carbone,

+ un radical cycloalkylène,

+ un radical alkénylène linéaire ou ramifié comportant 1 ou 2 doubles liaisons et ayant 1 à 24 atomes de carbone,
+ un radical alkylène comportant de 6 à 20 atomes de carbone, de 1 à 6 enchaînements constitués par - O -, - CO - ou - COO - et de 0 à 2 radicaux cyclo alkylène,

. $R_1$ et $R_2$, identiques ou différents possèdent des significations indiquées précédemment,
. l'un des cycles peut être partiellement ou totalement hydrogéné en cyclohexyle, cyclohexényle ou cyclohexadiényle ;

- les composés aromatiques de formule générale (III) :

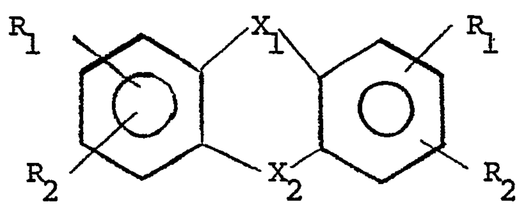

(III)

dans laquelle :

. les symboles $X_1$ et $X_2$ identiques ou différents représentent un lien valentiel simple, un atome d'oxygène ou atome de soufre ou un groupement méthylène,
. les symboles $R_1$ et $R_2$ identiques ou différents possèdent les significations indiquées précédemment,
. l'un des cycles peut être partiellement ou totalement hydrogéné ;

- les composés aromatiques hétérocycliques ayant un cycle ou 2 cycles condensés comportant 1 ou 2 hétéroatomes azote, soufre ou oxygène.

10) Procédé selon l'une des revendications 1 à 9 caractérisé en ce que le composé aromatique est choisi parmi :

- Les composés aromatiques de formule (I) dans laquelle $R_1$, $R_2$ et $R_3$, identiques ou différents, représentent :

. un atome d'hydrogène,
. un radical alkyle linéaire ou ramifié ayant de 1 à 12 atomes de carbone,

. un radical alkényle, linéaire ou ramifié, comportant 1 ou 2 doubles liaisons éthyléniques et ayant 2 à 12 atomes de carbone,

. un radical alkoxy, linéaire ou ramifié ayant 1 à 4 atomes de carbone,

. un atome de chlore, de fluor ou de brome,

. un groupement hydroxyle,

. un radical alkyle, linéaire ou ramifié, ayant de 2 à 12 atomes de carbone et comportant dans son enchaînement 1 ou 2 ponts - O -,

. un groupement $- O - R_4 - COOR_5$ dans la formule duquel $R_4$ représente un radical alkylène linéaire ou ramifié ayant 1 à 4 atomes de carbone et $R_5$ représente un radical alkyle linéaire ou ramifié ayant 1 à 8 atomes de carbone,

. un groupement $- COOR_6$ , dans la formule duquel $R_6$ représente un atome d'hydrogène ou un radical alkyle, linéaire ou ramifié, ayant 1 à 12 atomes de carbone,

. un groupement $- CO - R_6$ dans la formule duquel $R_6$ a les significations indiquées précédemment,

. un groupement $- O - CO - R_7$, dans la formule duquel $R_7$ représente un radical alkyle, linéaire ou ramifié, ayant 1 à 12 atomes de carbone,

. un groupement nitrile,

. un groupement 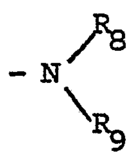 dans la formule duquel $R_8$ et $R_9$, identiques ou différents, représentent un atome d'hydrogène ou un radical alkyle linéaire ou ramifié ayant de 1 à 6 atomes de carbone,

. un groupement $- NH - CO - O - R_{10}$ dans la formule duquel $R_{10}$ représente un radical alkyle, linéaire ou ramifié ayant de 1 à 6 atomes de carbone,

. un groupement $- S - R_8$, dans la formule duquel $R_8$ a les significations indiquées précédemment,

- le naphtalène, le naphtalène comportant sur chacun de ses cycles condensés 1 ou 2 substituants $R_1$ et $R_2$ définis précédemment, le tétrahydro-1,2,3,4 naphtalène et le tétrahydro-1,2,3,4 naphtalène

comportant sur chacun de ses cycles 1 ou 2 substituants $R_1$ et $R_2$ définis précédemment,

- les composés aromatiques bicycliques de formule générale (II) : dans laquelle :

. Z représente : + un lien valentiel simple
+ un groupement - $SO_2$ -
+ un groupement - SO -
+ un atome d'oxygène
+ un atome de soufre,
+ un radical alkylène linéaire ou ramifié ayant 1 à 12 atomes de carbone,
+ un radical
$- O - R_{11} - O- CO - R_{12} - CO - O - R_{13} - O -$
dans la formule duquel $R_{11}$, $R_{12}$ et $R_{13}$, identiques ou différents, représentent des radicaux alkylènes linéaires ou ramifiés ayant 1 à 12 atomes de carbone, $R_{12}$ représentant en outre un radical cyclohexylène ou cyclohexanediméthylène-1,4 ,
+ un radical
$- O - R_{11} - COO - R_{12} - OCO - R_{13} - O -$
dans la formule duquel $R_{11}$, $R_{12}$ et $R_{13}$, identiques ou différents, ont les significations indiquées précédemment,

. $R_1$ et $R_2$, identiques ou différents possèdent les significations indiquées précédemment,

. l'un des cycles peut être un groupement cyclohexyle ;

- les composés aromatiques de formule générale (III) dans laquelle :

. les symboles $X_1$ et $X_2$ identiques ou différents représentent un lien valentiel simple ou un atome d'oxygène,

. les symboles $R_1$ et $R_2$, identiques ou différents, possèdent les significations indiquées précédemment,

. l'un des cycles peut être un groupement cyclohexyle ;

- les composés aromatiques hétérocycliques ayant 1 ou 2 cycles condensés et comportant 1 atome d'azote, de soufre ou d'oxygène.

11) Procédé selon l'une des revendications 1 à 10 caractérisé en ce que le composé aromatique est choisi dans le groupe comprenant : le benzène, le toluène, le monochlorobenzène, le méthoxybenzène, l'éthoxybenzène, l'orthoxylène, le métaxylène, le paraxylène, le monobromobenzène, le monofluorobenzène, le triméthyl-1,3,5 benzène, le triméthyl-1,2,4 benzène, le triméthyl-1,2,3 benzène, le triéthyl-1,2,4 benzène, le triéthyl-1,3,5 benzène, le diméthoxy-1,2 benzène, le diméthoxy-1,3 benzène, le diméthoxy-1,4 benzène, le difluoro-1,2 benzène, le difluoro-1,3 benzène, le difluoro-1,4 benzène, le dichloro-1,2 benzène, le dichloro-1,3 benzène, le dichloro-1,4 benzène, le phénoxy-2 acétate d'éthyle, le phénoxy-3 propionate d'éthyle, le phénoxy-4 butyrate d'éthyle, le phénoxy-2 acétate de butyle, le phénol, le méthoxy-2 phénol, le méthoxy-4 phénol, le benzoate de méthyle, le benzoate de butyle, le benzoate d'éthyle, le benzoate d'hexyle, le benzoate de dodécyle, l'acétophénone, la butyrophénone, l'hexanophénone, l'acétate de phényle, le propionate de phényle, le butyrate de phényle, l'octanoate de phényle, le benzonitrile, le diméthylaminobenzène, le diéthylaminobenzène, l'aniline, le phénylcarbamate d'éthyle, le phénylcarbamate de butyle, le phénylcarbamate d'hexyle, le thiophénol, le méthylthiobenzène, l'éthylthiobenzène, le butylthiobenzène ;

le naphtalène, le tétrahydro-1,2,3,4 naphtalène, le méthyl-1 naphtalène, le méthyl-2 naphtalène, l'éthyl-1 naphtalène, l'éthyl-2 naphtalène, le méthoxy-1 naphtalène, le méthoxy-2 naphtalène, l'éthoxy-1 naphtalène, l'éthoxy-2 naphtalène, le naphtol-1, le naphtol-2, le diméthyl-1,4 naphtalène, le diméthyl-2,3 naphtalène, le diméthoxy-1,4 naphtalène, le diméthoxy-1,3 naphtalène, le diméthoxy-1,2 naphtalène, le diméthoxy-2,3 naphtalène, le propoxy-1 naphtalène, le propoxy-2 naphtalène, le butoxy-1 naphtalène, le butoxy-2 naphtalène, le chloro-1 naphtalène, le chloro-2 naphtalène, le fluoro-1 naphtalène, le fluoro-2 naphtalène, le dichloro-1,2 naphtalène, le dichloro-1,3 naphtalène, le dichloro-1,4 naphtalène, le dichloro-1,5 naphtalène, le dichloro-1,6 naphtalène, le dichloro-1,7 naphtalène, le dichloro-1,8 naphtalène, le dichloro-2,3

naphtalène, le dichloro-2,6 naphtalène, le dichloro-2,7 naphtalène, le triméthyl-1,2,5 naphtalène, le triméthyl-1,2,6 naphtalène, le triméthyl-1,2,7 naphtalène, le triméthyl-2,3,6 naphtalène, le diméthyl-1,6 isopropyl-4 naphtalène, l'acétonaphtone, la propionaphtone, la butyronaphtone ;
le biphényle, le diphénylméthane, le diphényl-2,2 propane, le diphényl-1,3 diméthyl-2,2 pentane, l'oxyde de diphényle, le diphényl-1,6 hexane, le diphényl-1,8 octane, le diphényl-1,10 décane, le diphénylsulfoxyde, la diphénylsulfone, le sulfure de diphényle, le di(phénoxy-2 acétate) d'éthylèneglycol, le di(phénoxy-2 acétate) de butanediol-1,4, le di(phénoxy-2 acétate) de diméthyl-2,2 propanediyle, le di(phénoxy-3 propionate) d'éthylèneglycol, le succinate de di-(phénoxy-2 éthyle), l'adipate de di(phénoxy-2 éthyle), le sébaçate de di(phénoxy-2 éthyle), le succinate de di(phénoxy-3 propyle), l'adipate de di(phénoxy-3 propyle), le succinate de di(phénoxy-4 butyle), l'adipate de di(phénoxy-4 butyle), le di(phénoxy-2 acétate) de cyclohexanediméthylène-1,4 ;
le dibenzo-p-dioxane, le dibenzofuranne, le biphénylène et le fluorène ;
la pyridine, le thiophène, le furanne, le pyrrole, la quinoléine, l'isoquinoléine.

12) Procédé selon l'une des revendications 1 à 11 caractérisé en ce que le rapport pondéral acide fluorhydrique/composé aromatique est compris entre 1 et 20 et de préférence entre 3 et 10.

13) Procédé selon l'une des revendications 1 à 12 caractérisé en ce qu'il est mis en oeuvre entre -40°C et +50°C et de préférence entre -30°C et +30°C.

14) Utilisation des composés ß-dicétoniques obtenus selon l'une des revendications 1 à 13 pour la stabilisation des polymères halogénés et notamment du polychlorure de vinyle.

Office européen des brevets

**RAPPORT DE RECHERCHE EUROPEENNE**

0193475

Numero de la demande

EP 86 42 0029

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int. Cl.4) |
|---|---|---|---|
| D,Y | FR-A-2 238 696 (HOECHST AG)<br>* Revendications *<br>--- | 1 | C 07 C 45/76<br>C 07 C 45/46<br>C 07 C 45/57<br>C 07 C 49/84<br>C 07 C 49/782<br>C 07 C 49/807<br>C 07 C 49/813<br>C 07 C 49/76 |
| Y | US-A-2 214 117 (A.B. BOESE)<br>* Revendications *<br>--- | 1,2 | |
| Y | US-A-2 463 742 (A.C. BYRNS)<br>* Revendications *<br>--- | 1,2 | |
| Y | US-A-2 453 619 (A.C. BYRNS)<br>* Colonne 3; revendications *<br>--- | 1,2 | |
| A | EP-A-0 084 742 (RHONE-POULENC SPECIALITES CHIMIQUES)<br>* Revendications *<br>--- | 1 | DOMAINES TECHNIQUES RECHERCHES (Int. Cl.4) |
| A | EP-A-0 069 598 (RAYCHEM CORP.)<br>* Revendications *<br>----- | 1 | C 07 C 45/00<br>C 07 C 49/00<br>C 08 K 5/07 |

Le présent rapport de recherche a été établi pour toutes les revendications

| Lieu de la recherche | Date d'achèvement de la recherche | Examinateur |
|---|---|---|
| LA HAYE | 07-05-1986 | BONNEVALLE E.I.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur mais publié à la date de dépôt ou après cette date
D : cité dans la demande
L : cité pour d'autres raisons
& : membre de la même famille, document correspondant

OEB Form 1503 03.82